# EUROPEAN PATENT APPLICATION

(11) **EP 4 141 129 A1**
(43) Date of publication of application: **01.03.2023**
(21) Application number: 21793268.0
(22) Date of filing: 23.04.2021
(51) Int. Cl.: C12Q 1/70, C12Q 1/686

(54) **METHOD FOR DETECTING HBV GENOTYPE, OLIGONUCLEOTIDE AND KIT**

(30) Priority: 24.04.2020 CN 202010330948
(71) Applicant: Leadway (HK) Limited, Hong Kong (CN)
(72) Inventor: ZHANG, Taisong, Hangzhou, Zhejiang 310030 (CN); CHEN, Feifei, Hangzhou, Zhejiang 310030 (CN); DAI, Mingyan, Hangzhou, Zhejiang 310030 (CN); HONG, Yufeng, Hangzhou, Zhejiang 310030 (CN)
(74) Representative: Schiweck Weinzierl Koch Patentanwälte Partnerschaft mbB
(86) International application number: PCT/CN2021/089334
(87) International publication number: WO 2021/213503

(57) **Abstract**

The present invention provides a method, an oligonucleotide and a kit for detecting HBV genotypes, which by use of the fluorescent PCR technology, not only can determine the presence or absence of HBV DNA in a sample, but also can achieve the genotype identification of HBV genotypes A, B, C, D and C/D recombinant type that may be present in the sampl

## Description

### Field of the Invention

The present invention belongs to the fields of bioscience and biotechnology, and particularly relates to a method, an oligonucleotide and a kit for detecting HBV genotypes, which by use of the fluorescent PCR technology, not only can determine the presence or absence of HBV and its viral load in a sample, but also can achieve the genotype identification of HBV genotypes A, B, C, D and C/D recombinant type that may be present in the sample.

### Background of the Invention

Hepatitis B virus (HBV) belongs to Hepadnaviridae with a genome length of about 3.2 kb and has partially double-stranded circular DNA. As a specific hepatotropic virus, HBV DNA can also be detected in extrahepatic organ cells in recent years due to the progress in hybridization techniques for nucleic acid molecules. HBV DNA is composed of a negative strand (long strand) and a positive strand (short strand). Its positive strand has a replication function, but no transcription and translation functions. Its negative strand has four open reading frames (ORFs): an S gene region, consisting of the S gene, a pre-S1 segment (pre-S1) and a pre-S2 segment (pre-S2) which encode the surface antigen (HBsAg), pre-S1 and pre-S2 respectively; a C gene region for encoding the core antigen (HBcAg), and its carboxyl segment rich in arginine residues is hydrolyzed to produce a secreted e antigen (HBeAg); a P gene region for encoding the P protein that has DNA polymerase, reverse transcriptase and RNase H activities; and an X gene region for encoding the HBxAg protein that has the effect of trans-activating HBV genes, enhances the replication and expression of the HBV genes, and also can trans-activate intracellular proto-oncogenes, which may be associated with the occurrence of hepatocellular carcinoma.

HBV is the smallest DNA virus causing zoonosis and globally distributed. It can cause acute hepatitis, chronic hepatitis, cirrhosis and hepatitis B-associated liver cancer, posing an increased threat to human health. Based on nucleotide homology, HBV has been broadly divided into eight genotypes A to H (which can be abbreviated as A, B, C, D, E, F, G and H respectively), and can be subdivided into different subtypes within the same genotype. The distribution of HBV genotypes has a clear regional feature. In China, genotype C is predominant in the north and genotype B in the south; genotype A is the most prevalent all over the world and is more widely distributed in Northern Europe, North America and Central Africa, but has been occasionally reported in China; genotypes E and F have not yet been found in China; and in addition, genotype D is mainly distributed in the western regions of China such as Xinjiang and Xizang. In recent years, many literature reported that the western region of China is dominated by the C/D recombinant type, which is mainly divided into two subtypes: CD1 and CD2.

Different HBV genotypes are related to patients' clinical performance, prognosis and treatment response to a certain extent. Relevant studies indicate that the severity and activity of chronic hepatitis B disease are positively correlated with the level of viral replication, and the level of HBV DNA replication is obviously higher in genotype C than in genotype B. Therefore, compared with genotype B, patients suffered from genotype C are prone to chronicity, serve condition, and development of cirrhosis and liver cancer, and have a low antiviral response rate to interferon and poor prognosis. Therefore, HBV genotyping detection can provide a molecular biological reference for clinical laboratories to treat hepatitis B virus with antiviral drugs.

At present, common HBV genotyping detection methods include a fluorescent PCR method, a hybrid capture method and a PCR-reverse dot blot method. The hybrid capture method has disadvantages of no internal reference, long detection time (usually 5-6 hours), low sensitivity (3.5×10⁵ copies/ml), complicated operation, less detected genotypes and high false positive rate. Although the PCR-reverse dot blot method can detect relatively more HBV genotypes, it has disadvantages of long detection time (at least 4 hours), high detection cost, low detection sensitivity (10⁴ copies/ml), complicated operation, and failure of meeting the need of clinical high-throughput screening.

The fluorescent PCR method has high sensitivity and specificity, and can detect amplification products online in real time, thus it can significantly reduce the detection time and also avoid the occurrence of residual contamination. Common fluorescent PCR methods include an SYBR Green I dye method, a dual-probe hybridization method and a Taqman probe method. Among them, as SYBR Green I is an unsaturated dye, its specificity is not as good as those of the dual-probe hybridization method and the Taqman probe method, and must be judged by observing the melting curve; however, the cost of the dual-probe hybridization method is relatively expensive.

Currently, most of the reagents for HBV genotyping in the domestic market in China are dominated by detection of genotypes B and C; although there are few products that can detect genotype D, the existing reagents for detecting HBV genotypes cannot effectively distinguish genotype C, genotype D and the C/D recombinant type. Therefore, in the actual detection process, it often occurs that the HBV C/D recombinant type cannot be detected, or the C/D recombinant type is regarded as the case of genotype C or genotype D, which is not conducive to accurately guiding patients to use drugs and monitoring their disease progression and treatment prognosis. In addition, considering that there are still a small number of patients carrying a genotype other than genotypes A, B, C, and D and the C/D recombinant type, some detection products can detect genotypes A, B and C, and even genotype D, but cannot detect the presence or absence of other HBV genotypes, leading to missed detection.

### Summary of the Invention

In view of the shortcomings of the prior art, the present invention uses the fluorescent quantitative PCR method to perform HBV typing detection, which can not only detect genotypes A, B, C and D, but also distinguish the C/D recombinant type, in addition to adding the detection of HBV DNA, so that as long as HBV is present in the sample, it can be detected regardless of the genotype, so as to avoid missed detection.

An objective of the present invention is to provide an oligonucleotide for detection of HBV genotypes by fluorescent PCR, and the oligonucleotide comprises: (1) a first pair of primers and a first probe for specific detection of HBV genotype D and C/D recombinant type, the first pair of primers and the first probe being designed for the S gene region of the HBV genotype D; and (2) a second pair of primers and a second probe for specific detection of the HBV C/D recombinant type, the second pair of primers and the second probe being designed for the C gene region of the HBV C/D recombinant type.

Further, the first pair of primers and the first probe have base sequences of SEQ ID NOs: 13 to 15.

Further, the second pair of primers and the second probe have base sequences of SEQ ID NOs: 16 to 18.

Further, the oligonucleotide further comprises at least one of (3) to (6): (3) primers and a probe for detecting HBV genotype A, which have base sequences of SEQ ID NOs: 1 to 3 respectively; (4) primers and a probe for detecting HBV genotype B, which have base sequences of SEQ ID NOs: 4 to 6 respectively; (5) primers and a probe for detecting HBV genotype C, which have base sequences of SEQ ID NOs: 10 to 12 respectively; and (6) primers and a probe for detecting HBV DNA, which have base sequences of SEQ ID NOs: 7 to 9 respectively.

Further, the oligonucleotide further comprises: (7) primers and a probe for detecting an internal reference, which have base sequences of SEQ ID NOs: 19 to 21 respectively.

Another objective of the present invention is to provide a method for detecting HBV genotypes using fluorescent PCR. The method comprises: (1) extracting DNA from a sample; (2) performing fluorescent PCR amplification on the DNA in step (1) in the presence of a set of primers and probes; (3) determining whether the sample has the HBV genotype D or C/D recombinant type based on the result in step (2), wherein the set of primers and probes comprises a first pair of primers and a first probe for specific detection of the HBV genotype D and C/D recombinant type, and a second pair of primers and a second probe for specific detection of the HBV C/D recombinant type, the first pair of primers and the first probe being designed for the S gene region of the HBV genotype D, and the second pair of primers and the second probe being designed for the C gene region of the HBV C/D recombinant type. Further, the set of primers and probes further comprises a pair of primers and a probe for detecting HBV genotype C, which have base sequences of SEQ ID NOs: 10 to 12 respectively.

Further, the method further comprises performing fluorescent PCR amplification on the DNA in step (1) in the presence of another set of primers and probes, wherein the another set of primers and probes comprises at least one of 1) to 3): 1) primers and a probe for detecting HBV genotype A, which have base sequences of SEQ ID NOs: 1 to 3 respectively; 2) primers and a probe for detecting HBV genotype B, which have base sequences of SEQ ID NOs: 4 to 6 respectively; and 3) primers and a probe for detecting HBV DNA, which have base sequences of SEQ ID NOs: 7 to 9 respectively. Further, the first pair of primers and the first probe have base sequences of SEQ ID NOs: 13 to 15, and the second pair of primers and the second probe have base sequences of SEQ ID NOs: 16 to 18.

A still another objective of the present invention is to provide a kit for detecting HBV genotypes using fluorescent PCR. The kit comprises a fluorescent PCR reaction solution that comprises an oligonucleotide; the oligonucleotide comprises: (1) a first pair of primers and a first probe for specific detection of HBV genotype D and C/D recombinant type, which are designed for the S gene region of the HBV genotype D; and (2) a second pair of primers and a second probe for specific detection of the HBV C/D recombinant type, which are designed for the C gene region of the HBV C/D recombinant type.

Further, the first pair of primers and the first probe have base sequences of SEQ ID NOs: 13 to 15, and the second pair of primers and the second probe have base sequences of SEQ ID NOs: 16 to 18.

Further, the oligonucleotide further comprises at least one of (3) to (6): (3) primers and a probe for detecting HBV genotype A, which have base sequences of SEQ ID NOs: 1 to 3 respectively; (4) primers and a probe for detecting HBV genotype B, which have base sequences of SEQ ID NOs: 4 to 6 respectively; (5) primers and a probe for detecting HBV genotype C, which have base sequences of SEQ ID NOs: 10 to 12 respectively; and (6) primers and a probe for detecting HBV DNA, which have base sequences of SEQ ID NOs: 7 to 9 respectively.

Further, the oligonucleotide further comprises: (7) primers and a probe for detecting an internal reference, which have base sequences of SEQ ID NOs: 19 to 21 respectively.

A still further objective of the present invention is to provide a method for detecting HBV genotypes using fluorescent PCR, and the method comprises: (1) extracting DNA from a sample; (2) performing fluorescent PCR amplification on the DNA in step (1) in a first reaction tube in the presence of a first set of primers and probes; and (3) performing fluorescent PCR amplification on the DNA in step (1) in a second reaction tube in the presence of a second set of primers and probes; and (4) determining, based on the result in step (2), whether HBV DNA is present in the sample and whether HBV genotype A or B is present, and if HBV DNA is present, then determining, based on the result in step (3), whether HBV genotype C, D or C/D recombinant type is present in the sample, the first set of primers and probes having base sequences of SEQ ID NOs: 1 to 9, and the second set of primers and probes having base sequences of SEQ ID NOs: 10 to 18.

Beneficial effects: HBV genotyping detection using the fluorescent quantitative PCR method can not only detect genotypes A, B, C and D, but also distinguish genotypes C, D and the C/D recombinant type; and as the western regions of China have high HBsAg positivity rate and are relatively closed geographically, the distribution and clinical characteristics of the C/D recombinant type are well worth studying. The present invention also adds the detection of HBV DNA; as long as HBV is present in the sample, it can be detected regardless of the genotype, avoiding missed detection, and the total HBV viral load in the sample is also quantitatively detected; and in addition, when genotype A, B, C, D or C/D recombinant type is present, not only is the fluorescent PCR detection for a certain genotype positive, but also the detection for HBV DNA is positive, which can increase the reliability of the sample detection. The present invention also adds the detection of a non-competitive internal reference to monitor the detection process of the whole system, and thus can effectively prevent false negative results. The present invention takes UNGase + dUTP anti-contamination measures, which can effectively exclude false positive results. The primers and probes designed in the present invention are aimed at multiple gene regions of multiple HBV genotypes, instead of being limited in a single gene region, which helps avoid cross reaction and further perform genotype identification. The present invention can be used to perform HBV genotyping analysis on samples such as serum or plasma from a person who tests positive for HBV nucleic acid, which provides a molecular biological reference for clinical laboratories to select anti-HBV virus treatment regimens.

### Brief Description of the Drawings

Fig. 1 is a detection result of the FMA channel in PCR reaction solution 1.
Fig. 2 is a detection result of the VIC channel in PCR reaction solution 1.
Fig. 3 is a detection result of the ROX channel in PCR reaction solution 1.
Fig. 4 is a detection result of the CY5 channel in PCR reaction solution 1.
Fig. 5 is a detection result of the FMA channel in PCR reaction solution 2.
Fig. 6 is a detection result of the VIC channel in PCR reaction solution 2.
FIG. 7 is an detection result of the ROX channel in PCR reaction solution 2.
FIG. 8 is a detection result of the CY5 channel in PCR reaction solution 2.
Fig. 9 is a sensitivity detection result of the VIC channel for a genotype A positive sample in PCR reaction solution 1.
Fig. 10 is a sensitivity detection result of the FMA channel for a genotype B positive sample in PCR reaction solution 1.
Fig. 11 is a sensitivity detection result of the FMA channel for a genotype C positive in PCR reaction solution 2.
Fig. 13 is a sensitivity detection result of the ROX channel for a genotype D positive sample in PCR reaction solution 2.
Fig. 14 is a sensitivity detection result of the VIC channel for a C/D recombinant type positive sample in PCR reaction solution 2.
Fig. 15 is a sensitivity detection result of the ROX channel for a C/D recombinant type positive sample in PCR reaction solution 2.

### Detailed Description of the Embodiments

Given that different HBV genotypes are related to patients' clinical performance, prognosis and treatment response to a certain extent, it is necessary to perform HBV genotyping detection on an HBV positive sample. The present invention can detect not only HBV virus genotypes A, B, C and D but also the presence or absence of the C/D recombinant type by using the fluorescent quantitative PCR method, so that genotype C, genotype D and the C/D recombinant type can be distinguished.

The present invention also adds the detection of HBV DNA; as long as HBV is present in the sample, the presence of HVB DNA can be detected regardless of the genotype, avoiding missed detection; and in addition, when one or more of genotypes A, B, C, D and the C/D recombinant type are present in the clinical sample, not only is the fluorescent PCR detection for this one or more genotypes positive, but also the detection for HBV DNA is positive, which can increase the reliability of the sample detection.

In the present invention, HBV detection can be performed in two tubes: the first reaction tube can be used for detecting genotype A, genotype B and HBV DNA, the second reaction tube can be used for detecting genotype C, genotype D and the C/D recombinant type, and of course, the objects to be detected in each reaction tube can be appropriately adjusted according to the actual needs. In addition, a non-competitive internal reference is added to each of the first and second reaction tubes to monitor the detection process of the whole system, so that the occurrence of false negative results can be effectively prevented. The non-competitive internal reference added into the first and second reaction tubes can be either the same or different.

In the present invention, in order to reduce possible non-specific amplification generated during fluorescent PCR amplification as far as possible, an anti-Taq DNA polymerase antibody can be added to a fluorescent PCR reaction solution, and as this antibody can bind the active sites of Taq DNA polymerase, Taq DNA polymerase cannot play its function; and only in the denaturation phase, the anti-Taq DNA polymerase antibody is inactivated at high temperature and cannot bind to the active sites of Taq DNA polymerase, thereby enabling Taq DNA polymerase to play a catalytic role to synthesize new DNA strands.

The design of the primers and probes of the present invention and its genotyping idea are as follows: the primers and probes designed in the present invention are aimed at selecting multiple gene regions of multiple HBV genotypes, instead of being limited in a single gene region, which helps avoid cross reaction and perform further genotype identification. Specifically, a pair of primers and a probe for detecting HBV DNA are designed for conserved regions of HBV genotypes A to H, and primers and probes for detecting a HBV genotype are designed for specific fragments in the S gene region of a specific HBV genotype; however, given that the C/D recombinant type has partial sequence overlap with genotypes C and D, specific primers and probes designed according to the S gene region of genotype C can detect only genotype C, but not genotype D and the C/D recombinant type. The specific primers and probes designed according to the S gene region of genotype D can detect not only genotype D but also the C/D recombinant type, thus there is a problem that the C/D recombinant type is misdiagnosed as genotype D. Therefore, in order to accurately distinguish genotype D from the C/D recombinant type, the present invention additionally designs a pair of primers and a probe for the C gene region of the C/D recombinant type to distinguish genotype D from the C/D recombinant type.

For the sake of facilitating illustration, the primers and probes given in Table 1 are taken as examples for illustration. In this way, in the first reaction tube, fluorescent PCR amplification is performed on a clinical sample in four colors to detect HBV genotype A (VIC channel), HBV genotype B (FAM channel), HBV DNA (ROX channel), and internal reference (Cy5 channel), so as to not only detect the presence or absence of HBV in the sample, but also determine the presence or absence of genotype A or genotype B. In the second reaction tube, fluorescent PCR amplification and HBV genotyping detection are performed on the clinical sample in four colors to detect genotype C (FAM channel), genotype D and C/D recombinant type (VIC channel), C/D recombinant type (ROX channel), and reference gene (Cy5 channel), so as to not only detect the presence or absence of genotype C and genotype D in the sample, but also determine the presence or absence of the C/D recombinant type. In the second reaction tube, the VIC channel and the ROX channel can be used to determine the presence or absence of genotype D and the C/D recombinant type. Specifically, given that genotype C, genotype D and the C/D recombinant type have partial sequence overlap, the primers and probe for detecting genotype C can detect only genotype C but not the C/D recombinant type, but the primers and probe for detecting genotype D (VIC channel) can detect not only genotype D but also the C/D recombinant type. Therefore, an additional pair of primers and a probe are designed in the C gene region of the C/D recombinant type to distinguish genotype D from the C/D recombinant type (ROX channel): when both the VIC channel and the ROX channel are positive, it is determined that the C/D recombinant type is present; when the VIC channel is positive but the ROX channel is negative, it is determined that genotype D is present and the C/D recombinant type is absent; and when both the VIC channel and the ROX channel are negative, it is determined that genotype D and the C/D recombinant type are absent. For the other situation in the second reaction tube, i.e., it is impossible that the VIC channel is a negative and the ROX channel is positive, this is because the primers and probe (VIC channel) for detecting genotype D can detect not only genotype D but also the C/D recombinant type, and when the VIC channel is negative, which means the absence of both genotype D and the C/D recombinant type in the sample, at this point, the ROX channel is also negative and impossible to be positive, so there is no need to make further result determination for the ROX channel.

**Table 1 Primers and probes**

| Primers and probes | Sequence number | Base sequences (5'→3') | Notes |
|---|---|---|---|
| Primer1-F | SEQ ID NO:1 | GATTATCAAGGTATGTTGCCCG | Detecting genotype A |
| Primer1-R | SEQ ID NO:2 | TGCCTTGAGCAGGAGTCGT | |
| Probe1 | SEQ ID NO:3 | VIC-ACAACAACAACCAGTACG-MGB | |
| Primer2-F | SEQ ID NO:4 | AACACCCGTGTGTCTTGGC | Detecting genotype B |
| Primer2-R | SEQ ID NO:5 | TAACCAGGACAAATTGGAGGA | |
| Probe2 | SEQ ID NO:6 | FAM-CCAAATCTCCAGTCACT-MGB | |
| Primer3-F | SEQ ID NO:7 | CTAGGACCCCTGCTCGTGT | HBV DNA |
| Primer3-R | SEQ ID NO:8 | GAGAGAAGTCCACCACGAGTCTA | |
| Probe3 | SEQ ID NO:9 | ROX-CAGGCGGGGTTTTTCTTGTTGAC-B HQ2 | |
| Primer4-F | SEQ ID NO:10 | CTCGTGGTGGACTTCTCTCAAT | Detecting genotype C |
| Primer4-R | SEQ ID NO:11 | GTTGGGGACTGCGAATTTT | |
| Probe4 | SEQ ID NO: 12 | FAM-CACCCAMGTGTCCTG-MGB | |
| Primer5-F | SEQ ID NO: 13 | CTCGTGGTGGACTTCTCTCAAT | Genotype D, C/D recombinant type |
| Primer5-R | SEQ ID NO: 14 | GTTGGGGACTGCGAATTTT | |
| Probe5 | SEQ ID NO: 15 | VIC-ACACACGGTAGTTCC-MGB | |
| Primer6-F | SEQ ID NO:16 | ATTTGGAGCTTCCGTGGAGTTA | C/D recombinant type |
| Primer6-R | SEQ ID NO:17 | GCAGTATGGTGAGGTGAACAATGT | |
| Probe6 | SEQ ID NO: 18 | ROX-CCTTCTATTCGAGATCTCCTCGACA CCG--BHQ2 | |
| Primer7-F | SEQ ID NO:19 | TGCAAGAAGGATCGTTGAAGCT | Reference gene |
| Primer7-R | SEQ ID NO:20 | CAACAGTCTGGCCTTTCAGCA | |
| Probe7 | SEQ ID NO:21 | Cy5-ATGAGGGCAGGCCGCTATGATGGAT- BHQ2 | |

The following examples further illustrate the present invention. These examples are not intended to limit the protection scope of the present invention, but provides further understanding of the present invention.

### Example 1: Extraction of sample DNA

On the Promotor^{®} NES-32 nucleic acid purifier from Acon Biotech (Hangzhou) Co., Ltd., clinical sample DNA was extracted using reagents in the nucleic acid (DNA) extraction kit (magnetic bead method) from Aeon Biotech (Hangzhou) Co., Ltd.
1) The reagent solutions shown in Table 2 were added to the 96-well plate in advance (this step could be omitted if pre-loaded plate reagents were used).

**Table 2 Nucleic acid extraction solution**

| 96-well reagent plate | | Reagent composition | | | |
|---|---|---|---|---|---|
| Column 1 | Column 7 | Lysis solution | 400µl | | |
| Column 2 | Column 8 | Washing solution 1 | | 600µl | |
| Column 3 | Column 9 | Washing solution 2 | | 600µl | |
| Column 4 | Column 10 | Washing solution 2 | | 600µl | |
| Column 5 | Column 11 | Magnetic bead solution | | | 100µl |
| Column 6 | Column 12 | Adding 75µl elution solution at first, and then adding 30µl of paraffin oil | | | |

2) 20µl of proteinase K was added to column 1 and column 7 of the 96-well plate in order at first; and then 200µl of the sample to be extracted was added.
3) The program on the NES-32 nucleic acid purifier was edited according to the steps shown in Table 3 and run:

**Table 3 Nucleic acid extraction steps**

| Steps | Hole position | Name | Waiting time (min) | Mixing time (min) | Magnetic attraction time (sec) | Mixing speed | Volume (µl) | Temperature state | Temperature (°C) |
|---|---|---|---|---|---|---|---|---|---|
| 1 | 5 | Transferring magnetic beads | 0 | 1 | 60 | Slow | 100 | Lysing | 90 |
| 2 | 1 | Lysing | 0 | 15 | 90 | Slow | 620 | Lysing | 90 |
| 3 | 2 | Washing 1 | 0 | 2 | 90 | Slow | 600 | Eluting | 90 |
| 4 | 3 | Washing 2 | 0 | 2 | 90 | Slow | 600 | Eluting | 90 |
| 5 | 4 | Washing 3 | 0 | 0 | 90 | Slow | 600 | Eluting | 90 |
| 6 | 6 | Eluting | 0 | 5 | 90 | Slow | 100 | Eluting | 90 |
| 7 | 2 | Discarding magnetic beads | 0 | 1 | 0 | Slow | 600 | Close | 0 |

4) After completion of the automated extraction, the eluted products in columns 6 and 12 were transferred to a 1.5ml centrifuge tube (a tip being inserted into the bottom of the tube) to obtain the extracted nucleic acid solution for use in subsequent experiments, or for later use by storage at -20°C ± 5°C.

### Example 2: steps of fluorescent PCR reaction

PCR reaction solution 1 for detecting HBV virus genotypes A and B and HBV DNA was prepared, the detection system being 40 µl. The detailed formulation of the PCR reaction solution 1 was as shown below:
PCR reaction solution 1: Tris-HCl (pH 8.9) 10mmol/L, KCl 50mmol/L, MgCl₂ 4mmol/L, dNTPs each 150umol/L, BSA 0.2µg/µl, Primer1-F 100nmol/L, Primer1-R 100nmol/L, Probe1 75nmol/L, Primer2-F 100nmol/L, Primer2-R 100nmol/L, Probe2 75nmol/L, Primer3-F 100nmol/L, Primer3-R 100nmol/L, Probe3 75nmol/L, Primer7-F 75nmol/L, Primer7-R 75nmol/L, and Probe7 50nmol/L.

Enzyme mixture: Taq DNA polymerase 3.81U/µl, UNG 0.224U/µl, and anti-Taq DNA polymerase antibody 0.63U/µl.

19.5µl of the PCR reaction solution 1 and 0.5µl of the enzyme mixture were fully mixed, and then 20µl of the resultant mixture was loaded into the first reaction tube and transferred to the sample processing region. 20µl of the nucleic acid solution obtained in example 1 was added to the first reaction tube, then the first reaction tube was tightly covered and transferred to the detection region for real-time fluorescent PCR amplification and detection.

PCR reaction solution 2 for detecting HBV virus genotypes C and D and the C/D recombinant type was prepared, the detection system being 40µl. The detailed formulation of the PCR reaction solution 2 was as shown below:
PCR reaction solution 2: Tris-HCl (pH 8.9) 10mmol/L, KCl 50mmol/L, MgCl₂ 4mmol/L, dNTPs each 150umol/L, BSA 0.2µg/µl, Primer4-F 100nmol/L, Primer4-R 100nmol/L, Probe4 75nmol/L, Primer5-F 100nmol/L, Primer5-R 100nmol/L, Probe5 75nmol/L, Primer6-F 100nmol/L, Primer6-R 100nmol/L, Probe6 75nmol/L, Primer7-F 75nmol/L, Primer7-R 75nmol/L, and Probe7 50nmol/L;
enzyme mixture: Taq DNA polymerase 3.81U/µl, UNG 0.224U/µl, and anti-Taq DNA polymerase antibody 0.63U/µl.
19.5µl of the PCR reaction solution 2 and 0.5µl of the enzyme mixture were fully mixed, and then 20µl of the resultant mixture was loaded into the second reaction tube and transferred to a sample processing region. 20µl of the nucleic acid solution obtained in example 1 was added to the second reaction tube, then the second reaction tube was tightly covered and transferred to the detection region for real-time fluorescent PCR amplification and detection.

The real-time fluorescent PCR procedure was as shown in Table 4.

### Example 4: Real-time fluorescent PCR reaction procedure

| Steps | Number of cycles | Temperature (°C) | Reaction Time (min : sec) |
|---|---|---|---|
| 1 | 1 | 50 | 02:00 |
| 2 | 1 | 95 | 03:00 |
| 3 | 40 | 94 | 00:15 |
| | | 60 | 00:30 |

FAM, VIC, ROX and Cy5 were selected during detection, and data acquisition was set at 60°C.

Various channels and result explanation were as shown in Table 5.

**Table 5 Various channels and result explanation**

| Number | Fluorescent channel setting | Explanation |
|---|---|---|
| PCR reaction solution 1 | VIC | Genotype A |
| | FAM | Genotype B |
| | ROX | HBV DNA |
| | Cy5 | Reference gene |
| PCR reaction solution 2 | FAM | Genotype C |
| | VIC | Genotype D, C/D recombinant type |
| | ROX | C/D recombinant type |
| | Cy5 | Reference gene |

### 1. Internal reference determination

The Ct value of the reference gene channel (Cy5) in each reaction tube should be less than 40 in sample detection, and there was an obvious S-shaped amplification curve. If the above requirements were met, result determination was proceeded according to the following steps, otherwise the experiment was considered invalid, and errors in terms of instruments, reagents and amplification conditions should be checked.

### 2. Determination of detection results

2.1 Determination of HBV DNA: if the Ct value detected in the ROX channel of the PCR reaction solution 1 should be less than 40 and there was an obvious S-shaped amplification curve, it means that the sample was positive for HBV DNA, and then genotyping result determination was proceeded; and if there was no Ct value detected in the ROX channel of the PCR reaction solution 1, it means that the sample was negative for HBV DNA or below the lower limit of detection of reagents.

### 2.2 HBV genotyping determination was as shown in Table 6.

**Table 6. Determination results of HBV typing**

| Reaction tube | Fluores cent channel | Detection results | | | | | |
|---|---|---|---|---|---|---|---|
| PCR reaction solution 1 | FAM | - | + | - | + | - | - |
| | VIC | + | - | - | - | - | - |
| PCR reaction solution 2 | FAM | - | - | + | + | - | - |
| | VIC | - | - | - | - | + | + |
| | ROX | / | / | / | / | - | + |
| Result determination | | Genotype A | Genotype B | Genotype C | Mixed B/C | Genotype D | C/D recombinant type |

| | | | | | | | |
|---|---|---|---|---|---|---|---|
| "-" indicates negative or below the lower limit of detection of the reagent, "+" indicates positive, "/" indicates no result determination, and "mixed B/C" indicates co-presence of genotype B and genotype C. | | | | | | | |

### Example 3: Detection of Clinical Samples

30 clinical samples were detected according to the methods in examples 1 and 2, and the detection results were as shown in Table 7.

**Table 7. Detection Results of Clinical Samples**

| Sample Number | Detection Results of the Present invention | | | | | | | | Clinical Detection Results |
|---|---|---|---|---|---|---|---|---|---|
| Detected Genes | PCR Reaction solution 1 | | | | PCR Reaction solution 2 | | | | |
| | FAM | VIC | ROX | CY5 | FAM | VIC | ROX | CY5 | |
| 1 | + | - | + | + | - | - | / | + | B |
| 2 | + | - | + | + | - | - | / | + | B |
| 3 | + | - | + | + | - | - | / | + | B |
| 4 | - | - | + | + | - | + | - | + | D |
| 5 | + | - | + | + | - | - | / | + | B |
| 6 | - | - | + | + | + | - | / | + | C |
| 7 | + | - | + | + | - | - | / | + | B |
| 8 | + | - | + | + | - | - | / | + | B |
| 9 | + | - | + | + | - | - | / | + | B |
| 10 | + | - | + | + | + | - | / | + | Mixed B/C |
| 11 | - | + | + | + | - | - | / | + | A |
| 12 | + | - | + | + | + | - | / | + | Mixed B/C |
| 13 | - | - | + | + | - | + | + | + | C/D recombinant type |
| 14 | + | - | + | + | + | - | / | + | Mixed B/C |
| 15 | - | - | + | + | + | - | / | + | C |
| 16 | + | - | + | + | - | - | / | + | B |
| 17 | + | - | + | + | - | - | / | + | B |
| 18 | - | - | + | + | + | - | / | + | C |
| 19 | - | - | + | + | + | - | / | + | C |
| 20 | - | - | + | + | + | - | / | + | C |
| 21 | + | - | + | + | - | - | / | + | B |
| 22 | - | - | + | + | - | + | + | + | C/D recombinant type |
| 23 | - | - | + | + | - | + | + | + | C/D recombinant type |
| 24 | - | - | + | + | + | - | / | + | C |
| 25 | - | - | + | + | + | - | / | + | C |
| 26 | - | - | - | + | - | - | / | + | - |
| 27 | - | - | - | + | - | - | / | + | - |
| 28 | - | - | - | + | - | - | / | + | - |
| 29 | - | - | - | + | - | - | / | + | - |
| 30 | - | - | - | + | - | - | / | + | - |

| | | | | | | | | | |
|---|---|---|---|---|---|---|---|---|---|
| Note: "+" indicates a positive result, "-" indicates negative or below the lower limit of detection of the reagent, and "/" indicates no result determination. | | | | | | | | | |

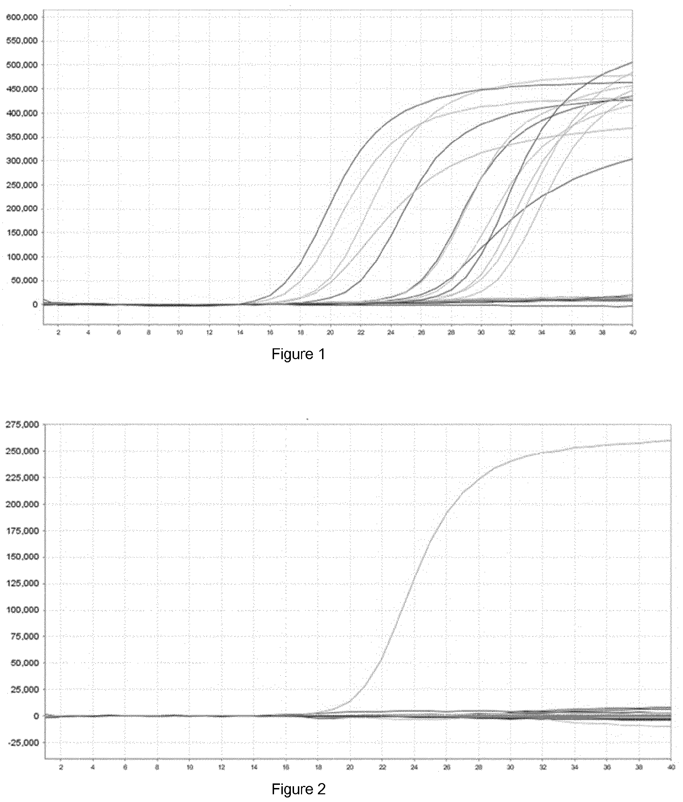

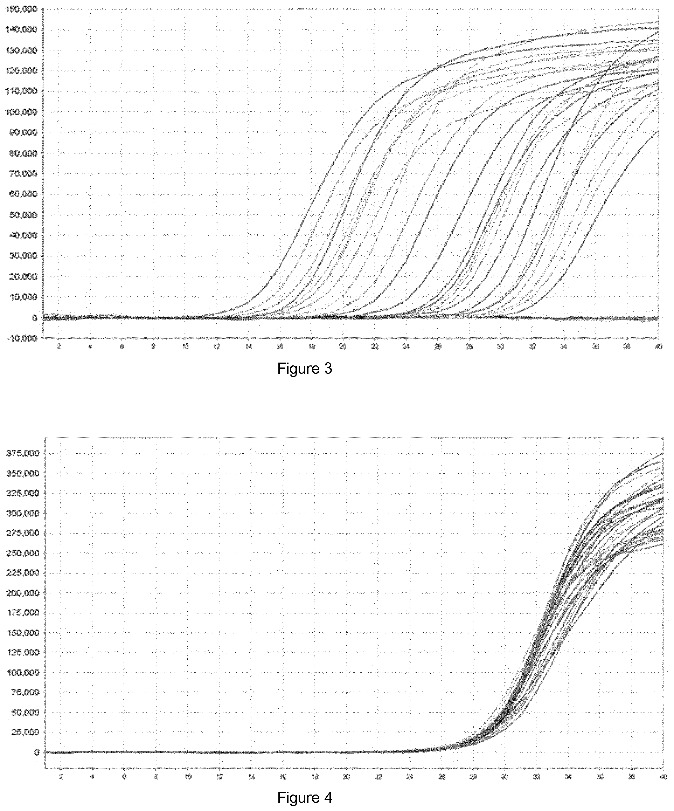

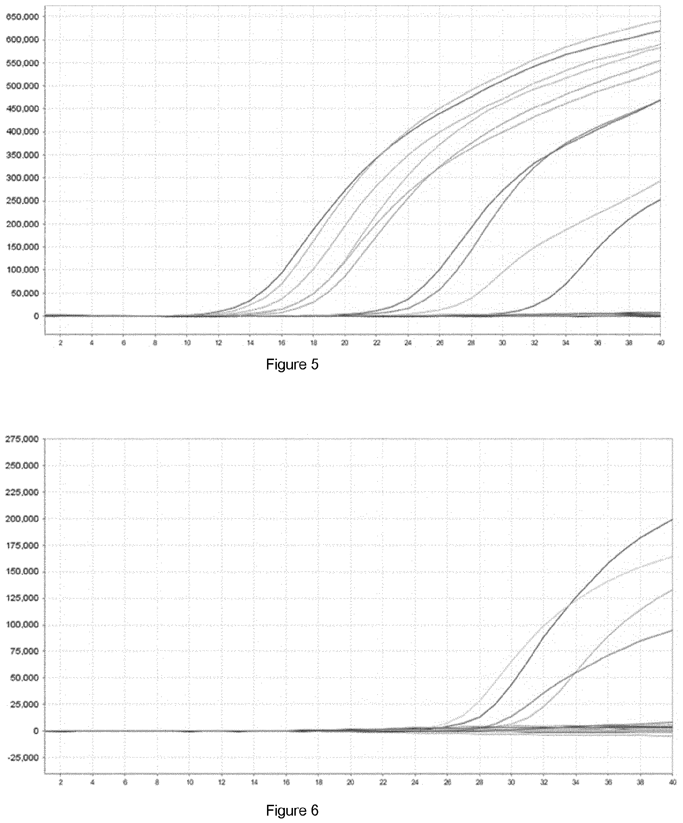

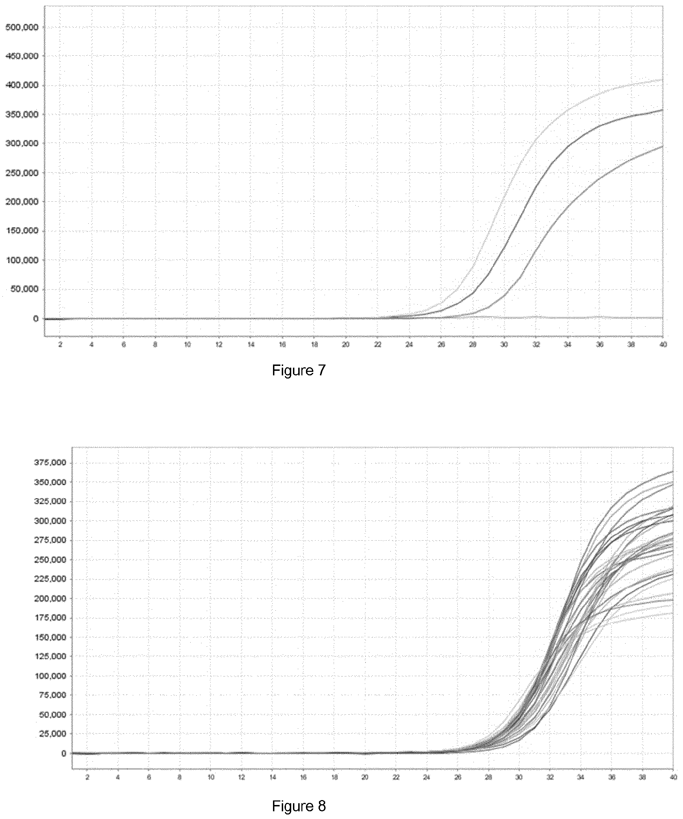

In these 30 samples, the results of the FAM, VIC, ROX and CY5 channels of the PCR reaction solution 1 were shown in Figs. 1 to 4 respectively; and the results of the FAM, VIC, ROX and CY5 channels of the PCR reaction solution 2 were shown in Figs. 5 to 8 respectively.

As shown in Figs. 1 to 8 and Table 7, in these 30 samples, there were 1 case of genotype A, 10 cases of genotype B, 7 cases of genotype C, 3 cases of mixed B/C, 1 case of genotype D, 3 cases of the C/D recombinant type and 5 cases of negative HBV. The detection results of the present invention were in line with the clinical detection results.

### Example 4: Detection Results of Sensitivity

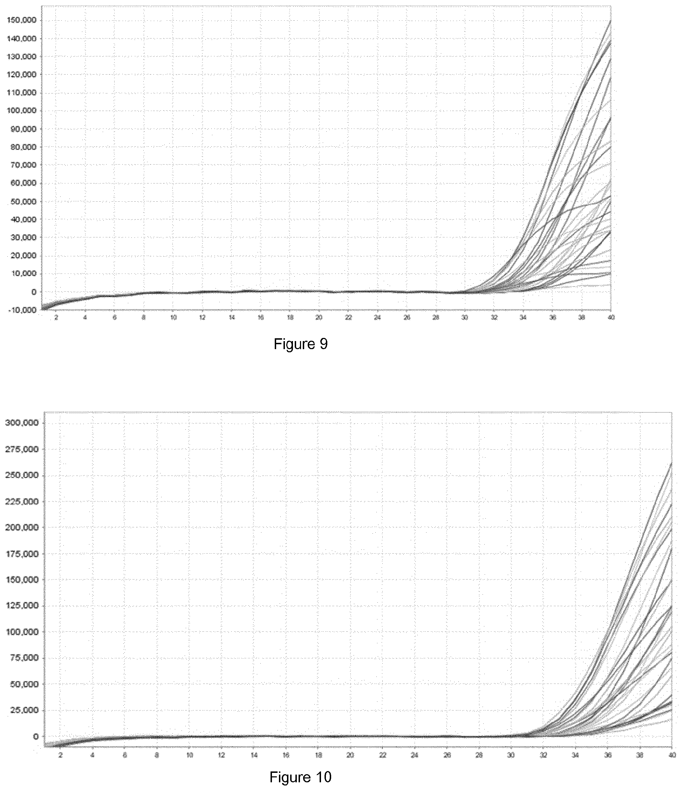

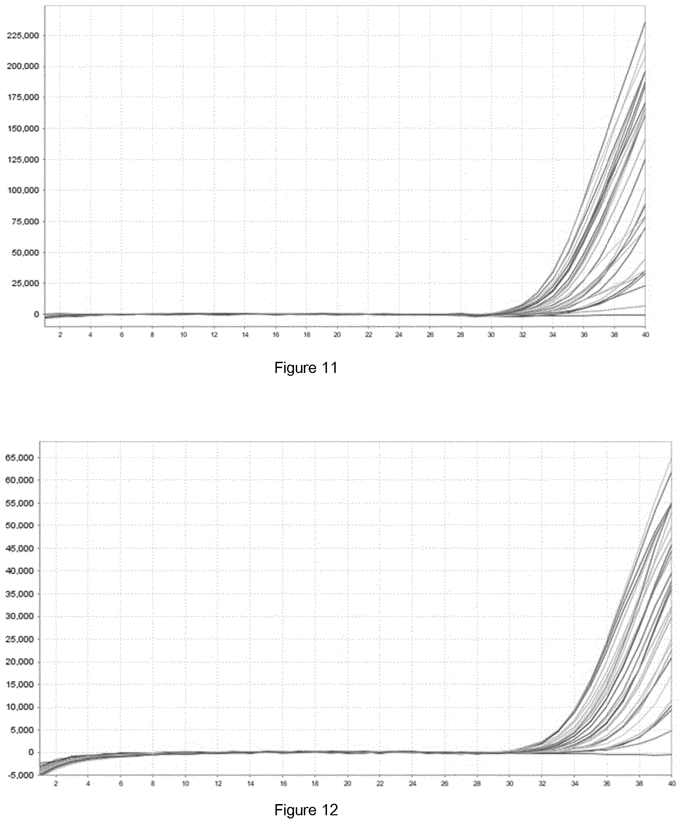

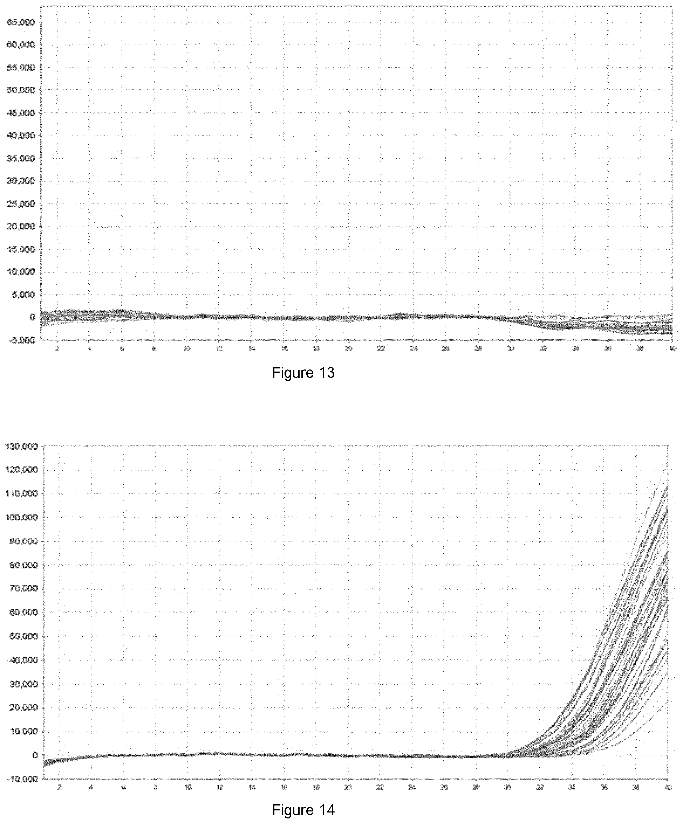

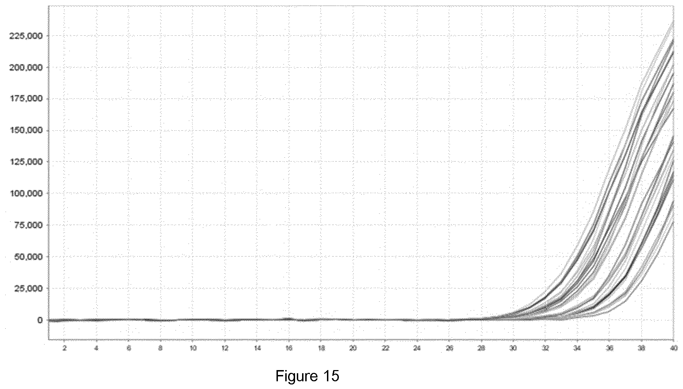

HBV genotype A, B, C, D, and the C/D recombinant type positive samples were detected respectively according to the methods of Examples 1 and 2. Each positive sample was subjected to gradient dilution to a concentration of about 100 IU/ml, 50 IU/ml, 20 IU/ml, and 10 IU/ml, respectively and each concentration was detected repeatedly 8 times. The detection results were shown in Table 8 and Figs. 9-15.

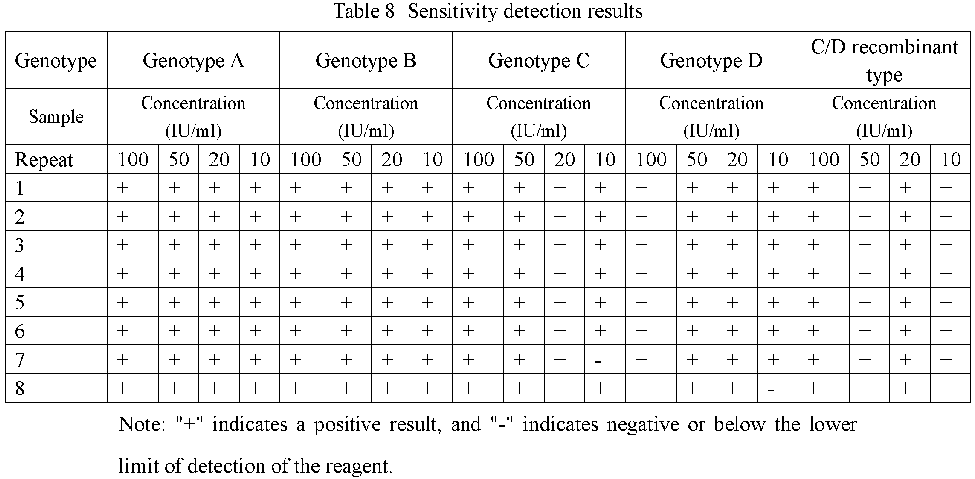

The experimental results showed that the detection sensitivity of the present invention was 10 IU/ml for genotype A, 10 IU/ml for genotype B, 20 IU/ml for genotype C, 20 IU/ml for genotype D, and 10 IU/ml for C/D recombinant type.

## Claims

1. An oligonucleotide for fluorescent PCR detection of HBV genotypes, comprising:
(1) a first pair of primers and a first probe for specific detection of HBV genotype D and C/D recombinant type, the first pair of primers and the first probe being designed for the S gene region of the HBV genotype D; and (2) a second pair of primers and a second probe for specific detection of the HBV C/D recombinant type, the second pair of primers and the second probe being designed for the C gene region of the HBV C/D recombinant type.

2. The oligonucleotide of claim 1, wherein the first pair of primers and the first probe have base sequences of SEQ ID NOs: 13 to 15.

3. The oligonucleotide of claim 1, wherein the second pair of primers and the second probe have base sequences of SEQ ID NOs: 16 to 18.

4. The oligonucleotide of claim 1, further comprising at least one of (3) to (6): (3) primers and a probe for detecting HBV genotype A, which have base sequences of SEQ ID NOs: 1 to 3 respectively; (4) primers and a probe for detecting HBV genotype B, which have base sequences of SEQ ID NOs: 4 to 6 respectively; (5) primers and a probe for detecting HBV genotype C, which have base sequences of SEQ ID NOs: 10 to 12 respectively; and (6) primers and a probe for detecting HBV DNA, which have base sequences of SEQ ID NOs: 7 to 9 respectively.

5. The oligonucleotide of any of claims 1 to 4, further comprising: (7) primers and a probe for detecting an internal reference, which have base sequences of SEQ ID NOs: 19 to 21 respectively.

6. A method for detecting HBV genotypes using fluorescent PCR, comprising: (1) extracting DNA from a sample; (2) performing fluorescent PCR amplification on the DNA in step (1) in the presence of a set of primers and probes; (3) determining whether the sample has the HBV genotype D or C/D recombinant type based on the result in step (2), wherein the set of primers and probes comprises a first pair of primers and a first probe for specific detection of the HBV genotype D and C/D recombinant type, and a second pair of primers and a second probe for specific detection of the HBV C/D recombinant type, the first pair of primers and the first probe being designed for the S gene region of the HBV genotype D, and the second pair of primers and the second probe being designed for the C gene region of the HBV C/D recombinant type.

7. The oligonucleotide of claim 6, wherein the set of primers and probes further comprises a pair of primers and a probe for detecting HBV genotype C, which have base sequences of SEQ ID NOs: 10 to 12 respectively.

8. The method of claim 6, further comprising performing fluorescent PCR amplification on the DNA in step (1) in the presence of another set of primers and probes, wherein the another set of primers and probes comprises at least one of 1) to 3): 1) primers and a probe for detecting HBV genotype A, which have base sequences of SEQ ID NOs: 1 to 3 respectively; 2) primers and a probe for detecting HBV genotype B, which have base sequences of SEQ ID NOs: 4 to 6 respectively; and 3) primers and a probe for detecting HBV DNA, which have base sequences of SEQ ID NOs: 7 to 9 respectively.

9. The method of claim 6, wherein the first pair of primers and the first probe have base sequences of SEQ ID NOs: 13 to 15, and the second pair of primers and the second probe have base sequences of SEQ ID NOs: 16 to 18.

10. A kit for detecting HBV genotypes using fluorescent PCR, comprising a fluorescent PCR reaction solution that comprises an oligonucleotide, wherein the oligonucleotide comprises: (1) a first pair of primers and a first probe for specific detection of HBV genotype D and C/D recombinant type, the first pair of primers and the first probe being designed for the S gene region of the HBV genotype D; and (2) a second pair of primers and a second probe for specific detection of the HBV C/D recombinant type, the second pair of primers and the second probe being designed for the C gene region of the HBV C/D recombinant type.

11. The kit of claim 10, wherein the first pair of primers and the first probe have base sequences of SEQ ID NOs: 13 to 15, and the second pair of primers and the second probe have base sequences of SEQ ID NOs: 16 to 18.

12. The kit of claim 10, wherein the oligonucleotide further comprises at least one of (3) to (6): (3) primers and a probe for detecting HBV genotype A, which have base sequences of SEQ ID NOs: 1 to 3 respectively; (4) primers and a probe for detecting HBV genotype B, which have base sequences of SEQ ID NOs: 4 to 6 respectively; (5) primers and a probe for detecting HBV genotype C, which have base sequences of SEQ ID NOs: 10 to 12 respectively; and (6) primers and a probe for detecting HBV DNA, which have base sequences of SEQ ID NOs: 7 to 9 respectively.

13. The kit of any of claims 10 to 12, wherein the oligonucleotide further comprises: (7) primers and a probe for detecting an internal reference, which have base sequences of SEQ ID NOs: 19 to 21 respectively.
